Europäisches Patentamt

European Patent Office

Office européen des brevets

(11) Veröffentlichungsnummer: **0 226 762**
**A1**

(12) **EUROPÄISCHE PATENTANMELDUNG**

(21) Anmeldenummer: **86115021.7**

(22) Anmeldetag: **29.10.86**

(51) Int. Cl.⁴: **A61F 2/34**

(30) Priorität: **18.11.85 CH 4914/85**

(43) Veröffentlichungstag der Anmeldung:
**01.07.87 Patentblatt 87/27**

(84) Benannte Vertragsstaaten:
**AT DE FR GB IT**

(71) Anmelder: **GEBRÜDER SULZER
AKTIENGESELLSCHAFT
Zürcherstrasse 9
CH-8401 Winterthur(CH)**
Anmelder: **Protek AG
Stadtbachstrasse 64
CH-3001 Bern(CH)**

(72) Erfinder: **Müller, Maurice E., Prof.Dr.-med
Melchenbühlweg 9
CH-3006 Bern(CH)**

(74) Vertreter: **Dipl.-Ing. H. Marsch Dipl.-Ing. K.
Sparing Dipl.-Phys.Dr. W.H. Röhl
Patentanwälte
Rethelstrasse 123
D-4000 Düsseldorf(DE)**

(54) **Künstliche Hüftgelenkspfanne.**

㉗ Die Pfannenschale (3) setzt sich zusammen aus einer Kugelkalotte (6) im Polbereich, die einen ersten Durchmesser (d) hat und aus aequatornahen Hohlkugelabschnitten (7), deren erzeugender Kreisbogen einen zweiten grösseren Durchmesser (D) hat. Beide Bereiche der Pfannenschale (3) - schneiden sich in einem Abstand (H) vom Scheitelpunkt der Pfannenschale (3). Der Abstand (H) beträgt 1/3 des ersten kleineren Durchmessers (d); der Durchmesser (d) ist durch den Durchmesser des zugehörigen Gelenkkopfes bestimmt und entspricht in seinem Absolutwert diesem zuzüglich einem Spiel, was mit Vorteil 0,5 mm beträgt.

Mit der neuen Schalenform wird das Auflagemuster des Gelenkkopfes in der Pfannenschale (3) verbessert; als mit geringem Spiel an den Gelenkkopf angepasste Auflagefläche wirkt dabei nur der Polbereich, wodurch die Gefahr eines Einklemmens des Gelenkkopfes vermindert wird. Die Hohlkugelabschnitte (7) mit grösserem Durchmesser (D) erlauben eine Führung des Gelenkkopfes bei einer Luxierung infolge starker Abwinkelung des Gelenkes, beispielsweise beim Sitzen; die Gefahr einer Luxation des Gelenkkopfes wird dadurch verringert.

## Künstliche Hüftgelenkspfanne

Die Erfindung betrifft eine künstliche Hüftgelenkspfanne, deren den Gelenkkopf aufnehmende, konkave Pfannenschale eine von der Kugelfläche abweichende, durch Kreisbogenstücke gebildete rotationssymmetrische Form hat, wobei die Mittelpunkte der Kreisbogenstücke im Abstand zueinander angeordnet sind.

Eine künstliche Hüftgelenkspfanne der genannten Art ist bekannt aus der CH-PS 593 054. Bei künstlichen Hüftgelenkspfannen, insbesondere wenn sie relativ weich d.h. in ihrer Elastizität an Elastizität des Beckenknochens angenähert sind, besteht einerseits das Problem des Bremstrommeleffektes, der sich im Laufe der Zeit infolge von,wenn auch minimalem,Abrieb noch verstärkt; andererseits ist eine Gefahr einer Luxation des Gelenkkopfes aus der Pfannenschale bei stark gebeugtem Gelenk,beispielsweise beim Sitzen gegeben. Unter "Bremstrommeleffekt" versteht man bekanntlich ein Einklemmen des Gelenkkopfes bei Verformungen der Pfanne und/oder der Pfannenschale, bei denen der freie Rand der Pfanne wie die Arme eines Greifers zusammenklappen; werden die Pfannenschalen zur Verminderung des Greifereffektes relativ flach ausgeführt, so besteht eine erhöhte Anfälligkeit der Gelenkprothese gegen Luxationen des Gelenkkopfes aus der Pfannenschale.

Zur Verminderung dieser Effekte ist die Pfannenschale der erwähnten bekannten Konstruktion einer Rotationsfläche, deren erzeugende Kurve aus zwei Kreisbogenteilen mit gleichen Radien zusammengesetzt ist, deren Mittelpunkte in der Aequatorebene liegend von der Rotationsachse einen horizontalen Abstand haben. In der Praxis hat sich gezeigt, dass ein Einklemmen des Gelenkkopfes mit der geschilderten bekannten Konstruktion, insbesondere nach längerer "Laufzeit" nicht in allen Fällen in ausreichendem Masse verhindert werden kann.

Aufgabe der Erfindung ist es daher eine Hüftgelenkspfanne zu schaffen, bei der ein Einklemmen des Gelenkkopfes in der Pfannenschale weitgehend vermieden ist, ohne dass die Gefahr von Luxationen erhöht wird. Diese Aufgabe wird mit der Erfindung dadurch gelöst, dass die Pfannenschale aus Kreisbogenstücken mit zwei verschiedenen Durchmessern gebildet ist, dass weiterhin ein Kreisbogenstück, dessen Mittelpunkt auf der Rotationssymmetrieachse in der Aequatorebene der Pfannenschale liegt mit einem ersten Durchmesser den Polbereich der Pfannenschale bildet, wobei dieser Polbereich eine Kugelkalotte darstellt, deren Höhe gleich 1/3 des ersten Durchmessers ist, dass ferner beidseitig des Polbereiches ein ringförmiger Hohlkugelabschnitt mit einem zweiten grösseren Durchmesser anschliesst, dessen Mittelpunkt auf der Rotationssymmetrieachse vom Pol weg aus der Aequatorebene verschoben ist.

Die auf den Polbereich beschränkte Anpassung der Schalenfläche an den Gelenkkopf, deren Durchmesser mit Vorteil gleich dem um ein Spiel von 0,5 mm vergrösserten Durchmesser des zugehörigen Gelenkkopfes ist, ergibt eine Begrenzung der Auflagefläche für den Gelenkkopf bei "normalen" Gelenkbewegungen auf diesen Bereich; in diesem ist eine "Greiferbewegung" seines "Randes" -wegen des erheblich kürzeren Hebelarmes gegenüber dem aequatorialen Rand -stark verringert. Bei einer Luxierung des Gelenkkopfes bei starken Beugen des Gelenkes bewirken die im aequatornahen Hohlkugelabschnitte eine Führung des Gelenkkopfes, so dass die Gefahr von Luxationen verringert wird. Der grössere Durchmesser dieser Abschnitte ergibt gleichzeitig ein vergrössertes Spiel zwischen Gelenkkopf und Pfannenschale im aequatornahen Bereich und damit eine zusätzliche Verminderung der Klemmgefahr.

Experimentell hat sich als günstig erwiesen, wenn der zweite Durchmesser das 1,02-fache bis 1,1-fache des ersten Durchmessers beträgt.

Die neue Pfanne kann generell aus allen bekannten Implantatwerkstoffen gefertigt werden; bevorzugte Materialien sind jedoch in bekannter Weise Polyäthylen oder Titan bzw. eine Titanlegierung. Weiterhin kann die neue Pfanne einteilig ausgeführt oder eine in einem äusseren Ring oder in einer Aussenschale eingebettete Innenschale einer zweiteiligen Gelenkpfanne bilden.

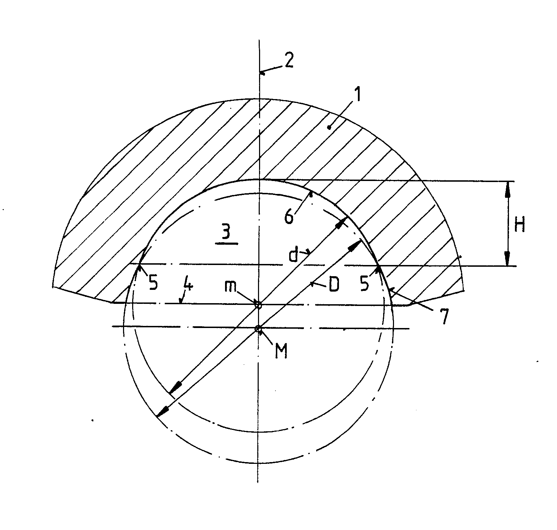

Im folgenden wird die Erfindung anhand eines Ausführungsbeispiels im Zusammenhang mit der Zeichnung näher erläutert.

Die einzige Figur zeigt schematisch einen Meredianschnitt der neuen Hüftgelenkspfanne.

Der nur schematisch dargestellte Pfannenkörper 1 hat eine Rotationssymmetrieachse 2 durch die sowohl seiner Aussenform als auch der Hohlraum der eigentlichen Pfannenschale 3 bestimmt sind.

Im Polbereich besteht die Pfannenschale 3 aus einer Kugelkalotte 6 mit einer Höhe H die gleich 1/3 ihres erzeugenden Kreisdurchmessers d ist. Der Mittelpunkt m der Kugelkalotte 6 bzw. des die erzeugenden Kreises liegt im Zentrum der Pfannenschale 3 wo die Rotationssymmetrieachse 2 die Aequatorebene 4 schneidet. Der Absolutwert des Durchmessers d entspricht demjenigen des zugehörigen nicht gezeigten Gelenkkopfes zuzüglich einem Spiel von 0,5 mm.

Im Abstand H vom Scheitelpunkt der Pfannenschale 3 weist die Schalenoberfläche eine Unstetigkeit 5 auf, die dadurch entsteht, dass die erzeugende der Oberfläche zwischen der Unstetigkeit 5 und der Aequatorebene 4 nunmehr ein Kreisbogenstück 7 mit einem etwas grösseren Durchmesser D ist. Der Durchmesser D beträgt im vorliegenden Beispiel das 1,065-fache des Durchmessers d.

Mit der Lage der Unstetigkeitsstelle 5 am Ende des Abstandes H vom Scheitelpunkt der Pfannenschale 3 und der Wahl des Durchmessers D ist die Lage des Mittelpunktes M für die Kreisbogenstücke 7 auf der Rotationssymmetrieachse 2 gegeben.

Der Pfannenkörper I kann auf seiner äusseren Oberfläche mit einer Strukturierung zur Verbesserung seiner Haftfähigkeit im Knochen versehen sein oder als Innenschale einer zweischaligen Pfanne dienen.

**Ansprüche**

I. Künstliche Hüftgelenkspfanne, deren den Gelenkkopf aufnehmende, konkave Pfannenschale eine von der Kugelfläche abweichende, durch Kreisbogenstücke gebildete rotationssymmetrische Form hat, wobei die Mittelpunkte der Kreisbogenstücke im Abstand zueinander angeordnet sind, dadurch **gekennzeichnet** , dass die Pfannenschale (3) aus Kreisbogenstücken mit zwei verschiedenen Durchmessern (d, D) gebildet ist, dass weiterhin ein Kreisbogenstück, dessen Mittelpunkt (m) auf der Rotationssymmetrieachse (2) in der Aequatorebene der Pfannenschale (3) liegt mit einem ersten Durchmesser (d) den Polbereich der Pfannenschale (3) bildet, wobei dieser Polbereich eine Kugelkalotte (6) darstellt, deren Höhe (H) gleich 1/3 des ersten Durchmessers (d) ist, dass ferner beidseitig des Polbereiches ein ringförmiger Hohlkugelabschnitt (7) mit einem zweiten grösseren Durchmesser (D) anschliesst, dessen Mittelpunkt (M) auf der Rotationssymmetrieachse (2) vom Pol weg aus der Aequatorebene (4) verschoben ist.

2. Hüftgelenkspfanne nach Anspruch I, dadurch gekennzeichnet, dass der zweite Durchmesser (D) das 1,02-fache bis 1,1-fache des ersten Durchmessers (d) beträgt.

3. Hüftgelenkspfanne nach Anspruch I oder 2, dadurch gekennzeichnet, dass der Absolutwert des ersten Durchmessers (d) gleich dem in mm gemessenen Durchmesser des zugehörigen Gelenkkopfes plus 0,5 mm ist.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A,D | FR-A-2 315 903 (SULZER) <br> * Ansprüche 1-4; Figur * | 1 | A 61 F  2/34 |
| | --- | | |
| A | EP-A-0 053 794 (FELDMÜHLE AG) <br> * Ansprüche 1,2; Figur 1 * | 1 | |
| | ----- | | |

RECHERCHIERTE SACHGEBIETE (Int. Cl.4)

A 61 F
F 16 C

Der vorliegende Recherchenbericht wurde für alle Patentansprüche erstellt.

| Recherchenort | Abschlußdatum der Recherche | Prüfer |
|---|---|---|
| DEN HAAG | 20-02-1987 | ERNST R.T. |